# EUROPEAN PATENT APPLICATION

(11) **EP 3 575 780 A1**
(43) Date of publication of application: **04.12.2019**
(21) Application number: 18745392.3
(22) Date of filing: 17.01.2018
(51) Int. Cl.: G01N 21/78

(54) **FECAL OCCULT BLOOD TEST PAD**

(30) Priority: 25.01.2017 CN 201710062804
(71) Applicant: Sigknow Biomedical Co., Ltd., Taipei City, Taiwan 10567 (CN)
(72) Inventor: CHOU, Wen-Pin, Taipei City 10567 (TW); WANG, Hsin-Yao, Taipei City 10567 (TW); WU, Min-Hsien, Taipei City 10567 (TW); WU, Chih-Liang, Taipei City 10567 (TW)
(74) Representative: Scholz, Volker
(86) International application number: PCT/CN2018/073029
(87) International publication number: WO 2018/137527

(57) **Abstract**

A test sheet for occult blood includes a permeable substrate layer, a test layer, and an outer layer. The permeable substrate layer and the test layer are jointed side by side or stacked from top to bottom on the outer layer. The permeable substrate layer is configured to carry biologic sample, and the test layer includes test agent. The outer layer protects the user from the biologic sample. At least one fold line is formed on the front surface of the connection of the permeable substrate layer and the test layer, or alternatively, on the front surface of the permeable substrate layer. When the test sheet is folded along the fold line, the biologic sample permeates the permeable substrate layer and reacts with the test agent. If the reaction causes color change, the biologic sample is determined to contain occult blood.

## Description

### CROSS-REFERENCE TO RELATED APPLICATION

This application claims the priority of China patent application No. 201710062804.8, filed on January 25, 2017, which is incorporated herewith by reference.

### BACKGROUND OF THE INVENTION

### 1. Field of the Invention

The present invention relates to a test sheet for occult blood, and more specifically to a test sheet provided for the user to bend and fold along at least one fold line to force the biological sample to diffuse into, contact, and react with the test agent such that it is possible to preliminarily identify the biological sample containing occult blood and further determine colorectal cancer developed if the reaction causes color change, thereby easily performing the process of screening potential colorectal cancer for ordinary people at home, and greatly increasing popularity of screening the disease.

### 2. The Prior Arts

Among top 10 cancers, colorectal cancer has a developing rate, which has recently increased to top 1 or 2 because of popularity of western diet and food risk. For the developing rate of colorectal cancer, it is a worldwide common topic to early screen and diagnose the disease. Fortunately, the develop process of colorectal cancer is generally slow. For example, it takes about ten years for intestine polypus with low risk to develop a cancer with high risk. It is possible to greatly decrease death rate caused by colorectal cancer if diagnosis and therapy are early performed. Thus, early diagnosis and therapy can not only save many lives, but also get rid of pain during normal therapy, and avoid considerable social cost.

Owing to many benefits resulting from early screening colorectal cancer, many screen methods have been exploited by employing chemistry, immunity, and colonoscopy. For the principle of chemistry for screening, it is well known that occult blood substantially contains hemoglobin, and iron ionic of hemoglobin may perform redox reaction with an external test agent. Thus, some specific color substance able to cause color change due to the reaction is employed to identify occult blood in feces. Similar to the above method of chemistry, the immunity method is also related to feces sampling and examination. Latex micro particles with specific antibody are used to detect hemoglobin in feces. If the feces contain occult blood, the specific antibody combines hemoglobin, and the turbidity method can quantitatively determine occult blood in feces based on turbidity of the test solution in the laboratory. In the viewpoint of economical therapy, the immunity method involving specific antibody is very high, and it is an extreme challenge for most laboratories to use and keep the test agent. In contract to the method of chemistry or immunity, the colonoscopy method directly uses colonoscopy to examine the wall of the large intestine. The benefit is that the doctor can also use colonoscopy to directly excise lesion or focus if any found. In other words, both diagnosis and therapy are performed at one time by colonoscopy. However, colonoscopy is an intrusive medical instrument, and it is reported that about 1 to 3 among 1,000 patients under treatment of colonoscopy every year would suffer serious comorbidity like large intestine rupture. It is concluded that the screen method of chemistry has the benefits such as excellent sensitivity and uniqueness, low cost, non-intrusive feature, and no comorbidity. Thus, the chemistry method is better than the method of immunity or colonoscopy for preliminarily screening colorectal cancer.

When it comes to the test procedure, the preset screen methods, however, causes some inconvenience and adversely affects popularity of screening the disease for ordinary people. The methods of chemistry and immunity are related to examination of feces. Specifically, the whole process comprises steps of sampling the feces, delivering the feces sample, examining the feces sample in the laboratory, and completing the test report. Frankly speaking, it is very complicated, and most patients often feel troublesome and uncomfortable to sample the feces. In addition, the process of delivery, examination, and report is tedious and inconvenient for ordinary people. As a result, people are not willing to accept the screen process for occult blood. As for the screen method of using colonoscopy, people need to go to hospital in person. In summary, popularity of screening colorectal cancer is adversely affected and greatly reduced because of difficult arrival, insufficiency of inspection instrument, and fewer gastroenterologists and anesthetists.

Therefore, it is greatly needed to provide a test sheet for occult blood which the user just bends and folds along at least one fold line to force the biological sample to diffuse into, contact, and react with the test agent so as to preliminarily identify occult blood by easily and simply wiping and folding the test sheet without any pre-treatment or post-treatment for the biological sample, thereby overcoming the problems in the prior arts.

### SUMMARY OF THE INVENTION

The primary objective of the present invention is to provide a test sheet for occult blood, comprising a permeable substrate layer, a test layer, and an outer layer. The permeable substrate layer is water permeable, and has a front surface for carrying a biologic sample like feces, urine, or biological excretions. The test layer is translucent and water permeable, and contains a test agent in at least one area. The outer layer is transparent, water impermeable, and attached to a back surface of the permeable substrate layer and a back surface of the test layer. In addition, one side of the permeable substrate layer is connected to one corresponding side of the test layer, and at least one fold line is formed on a front surface of a connection of the permeable substrate layer and the test layer.

Specifically, the biologic sample diffuses into the test layer after the at least one fold line is folded, and is further mixed with the test agent to proceed a predetermined reaction to cause color change for examining if the biologic sample contains occult blood.

Preferably, the permeable substrate layer comprises at least one of chemical fiber, plant fiber, animal fiber, and mineral fiber, and the test layer comprises at least one of plant fiber, nitride fiber, polyethylene (PE), polypropylene (PP), cellulose acetate fiber, polyvinylchloride (PVC) and polyvinylidenechloride (PVDC).

Preferably, the chemical fiber comprises at least one of polyester, nylon, spandex, acrylate, and cellulose acetate fiber, the animal fiber comprises at least one of animal hair, silk fiber, and spider fiber, and the mineral fiber comprises asbestos.

Another objective of the present invention is to provide a test sheet for occult blood, comprising a permeable substrate layer, a test layer, and an outer layer, which are sequentially stacked together from top to bottom. The permeable substrate layer is provided with water permeability, and has a front surface for carrying a biologic sample, and at least one fold line is formed on a middle region of the front surface for bending and folding. The test layer with translucency and water permeability has a front surface attached to the back surface of the permeable substrate layer. Further, the test layer comprises at least one chamber, and the chamber is provided with at least two compartments for accommodating a test agent. The at least two compartments is separated each other by a separate film, and the separate film is vertically aligned to the fold line. The test agent comprises a color substance and a redox substance, which are accommodated in the two different compartments, respectively. The outer layer like a hard film structure is provided with transparency and water impermeability, and attached to the back surface of the test layer.

The redox substance proceeds a redox reaction with an iron-based matter for decomposing and releasing free electrons, and the color substance possesses a function of receiving the free electrons released from the redox substance to cause color change.

The biological sample is sandwiched by the front surface of the permeable substrate layer after the fold line is folded, and then, the biological sample diffuses into the test layer. The separate film is broken and releases the color substance and the redox substance accommodated in the at least two compartments after the fold line is folded, and the color substance and the redox substance contact and react with the biological sample to cause color change for examining if the biologic sample contains occult blood.

Preferably, the chemical fiber comprises at least one of polyester, nylon, spandex, acrylate, and cellulose acetate fiber, the chemical fiber comprises at least one of polyester, nylon, spandex, acrylate, and cellulose acetate fiber, the animal fiber comprises at least one of animal hair, silk fiber, and spider fiber, and the mineral fiber comprises asbestos.

Preferably, the iron-based matter comprises an iron-based substance of hemoglobin in blood, the redox substance comprises at least one of hydrogen peroxide and sodium percarbonate (2NaCO₃·3H₂O), and the color substance comprises at least one of o-toluidine, phenolphthalein, pyramidon, guaiac, guaiac gum, tetramethylbenzidine, and organic alcohol.

A yet objective of the present invention is to provide a test sheet for occult blood, comprising a permeable substrate layer, a test layer, and an outer layer, which are sequentially stacked together from top to bottom. The permeable substrate layer is provided with water permeability, and has a front surface for carrying a biologic sample, and at least one fold line is formed on a middle region of the front surface for bending and folding. The test layer with translucency and water permeability has a front surface attached to the back surface of the permeable substrate layer. Further, the test layer comprises at least one chamber, and the chamber is provided with at least two compartments for accommodating a test agent. The at least two compartments is separated each other by a separate film, and the separate film is vertically aligned to the fold line. The test agent comprises a color substance and a redox substance, which are accommodated in the two different compartments, respectively. The test layer is provided with a plurality of connection passages, which are configured not to cross one another, and each connection passage is formed by extending the corresponding chamber toward the fold line. Moreover, the outer layer like a hard film structure is provided with transparency and water impermeability, and attached to the back surface of the test layer.

The redox substance possesses a function of proceeding a redox reaction with an iron-based matter for decomposing and releasing free electrons, and the color substance possesses a function of receiving the free electrons released from the redox substance to cause color change.

The biological sample is sandwiched by the front surface of the permeable substrate layer after the fold line is folded, and then, the biological sample diffuses into the test layer. The separate film is broken and releases the color substance and the redox substance accommodated in the at least two compartments after the fold line is folded, and the color substance and the redox substance contact and react with the biological sample to cause color change for examining if the biologic sample contains occult blood.

Preferably, the chemical fiber comprises at least one of polyester, nylon, spandex, acrylate, and cellulose acetate fiber, the chemical fiber comprises at least one of polyester, nylon, spandex, acrylate, and cellulose acetate fiber, the animal fiber comprises at least one of animal hair, silk fiber, and spider fiber, and the mineral fiber comprises asbestos. The test layer comprises at least one of plant fiber, nitride fiber, polyethylene, polypropylene, cellulose acetate fiber, polyvinylchloride and polyvinylidenechloride, the iron-based matter comprises an iron-based substance of hemoglobin in blood, the redox substance comprises at least one of hydrogen peroxide and sodium percarbonate, and the color substance comprises at least one of o-toluidine, phenolphthalein, pyramidon, guaiac, guaiac gum, tetramethylbenzidine, and organic alcohol. Further, the connection passage has a length configured not to cross the fold line.

Preferably, the connection passage has a length configured to cross the fold line, and the connection passage is provided with at least one notch structure configured for vertically aligning the fold line. Further, the at least one notch structure is broken after the fold line is folded, and the color substance and the redox substance accommodated in the at least two compartments are released through the at least one notch structure to diffuse, contact and react with the biological sample around the at least one notch structure.

The above connection passage has a length configured to cross the fold line or not to cross the fold line. If the connection passage is designed to cross the fold line, the connection passage is provided with at least one notch structure configured for vertically aligning the fold line. The at least one notch structure is specifically design to be broken after the fold line is folded, the color substance and the redox substance accommodated in the at least two compartments are released through the at least one notch structure to diffuse, contact and react with the biological sample around the at least one notch structure.

Therefore, the test sheet of the present invention has a simple structure and is very convenient to use without any pre-treatment or post-treatment for the biological sample. In particular, the user would not feel any discomfort, and the test sheet is very sanitary. As a result, overall efficiency of preliminarily examining occult blood is increased, and the overall operation is simple for ordinary people to perform alone at home without any specific assistance by medical personnel. It is obvious that the present invention indeed possesses industrial utility and market competitiveness.

### BRIEF DESCRIPTION OF THE DRAWINGS

The present invention will be apparent to those skilled in the art by reading the following detailed description of a preferred embodiment thereof, with reference to the attached drawings, in which:
FIG. 1 shows a test sheet for occult blood according to the first embodiment of the present invention;
FIG. 2 shows a test sheet for occult blood according to the second embodiment of the present invention;
FIG. 3 shows a test sheet for occult blood according to the third embodiment of the present invention; and
FIG. 4 shows a test sheet for occult blood according to the forth embodiment of the present invention.

### DETAILED DESCRIPTION OF THE PREFERRED EMBODIMENT

The accompanying drawings are included to provide a further understanding of the invention, and are incorporated in and constitute a part of this specification. The drawings illustrate embodiments of the invention and, together with the description, serve to explain the principles of the invention.

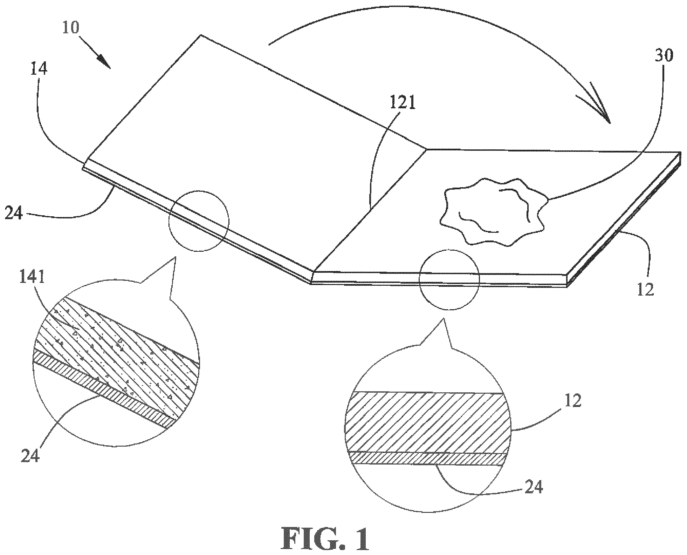

Please refer to FIG. 1, showing a test sheet for occult blood according to the first embodiment of the present invention. As shown in FIG. 1, the test sheet 10 for occult blood according to the first embodiment of the present invention generally comprises a permeable substrate layer 12, a test layer 14, and an outer layer 24. A back surface of the permeable substrate layer 12 and a back surface of the test layer 14 are configured to attach a front surface of the outer layer 24, and one side of the permeable substrate layer 12 is connected to one corresponding side of the test layer 14. Further, at least one fold line 121 is formed on a front surface of a connection of the permeable substrate layer 12 and the test layer 14. For clear description, FIG. 1 shows only one fold line 121. The permeable substrate layer 12 has a front surface for carrying a biologic sample 30 like feces, urine, or biological excretions.

Specifically, the permeable substrate layer 12 is water permeable such that the biologic sample 30 is easily attached by wiping. The permeable substrate layer 12 preferably comprises at least one of chemical fiber, plant fiber, animal fiber, and mineral fiber. The chemical fiber comprises at least one of polyester, nylon, spandex, acrylate, and cellulose acetate fiber, the animal fiber comprises at least one of animal hair, silk fiber, and spider fiber, and the mineral fiber comprises asbestos.

The test layer 14 contains a test agent 141 evenly distributed in at least one area of the test layer 14. For example, the test agent 141 is distributed only in the middle area as shown in FIG. 1 for simplicity. Specifically, the biologic sample 30 diffuses into the test layer 14 and further contact the test agent 141 so as to invoke a predetermined reaction. It is thus determined if the biologic sample 30 contains occult blood based on color change caused by the reaction. The function of examining occult blood in the biologic sample 30 is implemented. For instance, if the biologic sample 30 containing occult blood is feces, colorectal cancer is possibly developed. Moreover, the test layer 14 comprises at least one of plant fiber, nitride fiber, polyethylene (PE), polypropylene (PP), cellulose acetate fiber, polyvinylchloride (PVC) and polyvinylidenechloride (PVDC).

Since the above fold line 121 is located at the connection of the permeable substrate layer 12 and the test layer 14, it is easy to bend and fold along the fold line 121 to make the permeable substrate layer 12 and the test layer 14 in contact. At this time, the biologic sample 30 on the permeable substrate layer 12 thus contacts the test agent 141 in the test layer 14 to react.

The outer layer 24 is designed to prevent the user from touching the biological sample 30 for improving sanitation in use. Further, the outer layer 24 provides appropriate mechanical strength to avoid the test sheet 10 bending due to misuse. For example, the outer layer 24 is bent and folded only when an external force is greater than some threshold. In addition, since the outer layer 24 is somewhat transparent and particularly water impermeable, the user can easily examine if color change is caused by the biologic sample 30 and the test agent 141 mixed together through the back surface of the outer layer 24. Another function of the outer layer 24 is to improve convenience and sanitation in use. The user takes the back surface of the outer layer 24 by one hand and wipes the biological sample 30 such that the biological sample 30 is attached to the front surface of the permeable substrate layer 12. Then, the permeable substrate layer 12 and the test layer 14 contact each other after the back surface of the outer layer 24 is folded along the fold line 141 by asserting force such that the biological sample 30 is sandwiched and sealed between the permeable substrate layer 12 and the test layer 14 without exposure. Moreover, the biological sample 30 would not diffuse into the back surface of the outer layer 24 and not swear the user' hand because the outer layer 24 is water impermeable so as to get rid of any uncomfortable feeling.

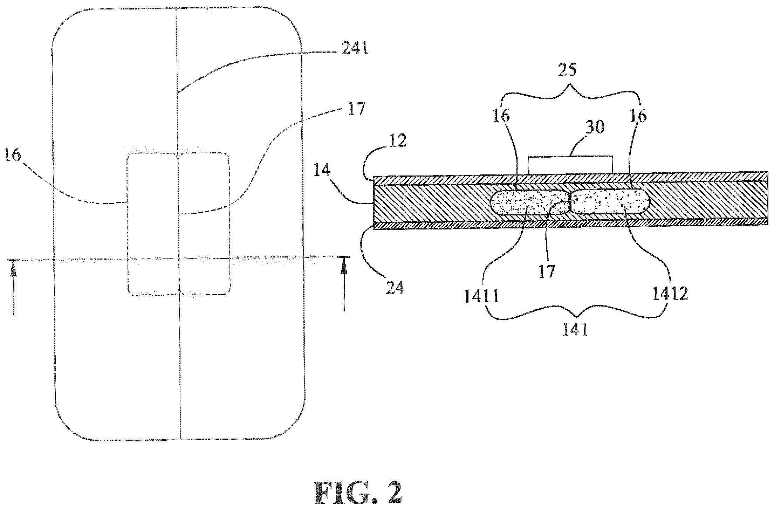

Further refer to FIG 2 illustrating the test sheet for occult blood according to the second embodiment of the present invention. The left-hand part in FIG. 2 is a top view of the test sheet, and the right-hand part in FIG. 2 is a side view. As shown in FIG. 2, the test sheet 10 of the second embodiment comprises a permeable substrate layer 12, a test layer 14, and an outer layer 24. The second embodiment is similar to the fist embodiment. For instance, the front surface of the permeable substrate layer 12 is also configured to carry the biological sample 30. However, one difference is that the permeable substrate layer 12, the test layer 14, and the outer layer 24 of the second embodiment are sequentially stacked together from top to bottom instead of the permeable substrate layer 12 and the test layer 14 of the first embodiment connected one side by one side and configured on the outer layer 24. Additionally, the front surface of the permeable substrate layer 12 is provided with at least one fold line 121 like the middle area in the permeable substrate layer 12 as shown in FIG. 2. However, only one fold line 121 is shown for simplicity.

More specifically, the test layer 14 of the second embodiment comprises at least one chamber 15. Only one chamber 15 is shown in FIG. 2 for clear description. Each chamber 15 has at least two compartments 16 for accommodating a test agent 141. For example, the at least two compartments 16 are located at the left and right hand side of the fold line 121, respectively. Further, the at least two compartments 16 are separated by a separate film 17, and the separate film 17 is vertically aligned to the fold line 121. It should be noted that only two compartments 16 and one separate film 17 are shown in FIG. 2. In other words, the present invention actually comprises at least one separate film 17 for separating two adjacent compartments 16. For instance, the compartments 16 are arranged in a matrix or a circle.

Further, the test agent 141 comprises a color substance 1411 and a redox substance 1412, which are accommodated in the left-hand compartment 16 and the right-hand compartment 16, respectively. The redox substance 1412 may perform a redox reaction with the iron-based substance (like iron ion contained in hemoglobin) in the biological sample 30, and then decompose. At the same time, free electrons are released to cause the color substance 1411 to change color, thereby implementing an effect of color change. Thus, if the biological sample 30 has color change after contacting the color substance 1411, it obviously means that the biological sample 30 possibly contain blood, that is, occult blood.

It is preferred that the color substance 1411 comprises at least one of o-toluidine, phenolphthalein, pyramidon, guaiac, guaiac gum, tetramethylbenzidine, and organic alcohol, and the redox substance 1412 comprises at least one of hydrogen peroxide and sodium percarbonate.

In actual applications, the user may take the outer layer 24 like the position aligning the left and right sides of the fold line 121, and then assert appropriate force onto the back surface of the outer layer 24 to bend and fold the whole test sheet 10 along the fold line 121 such that the left-hand side and the right-hand side of the front surface of the permeable substrate layer 12 with respect to the fold line 121 are folded and contact each other. At this time, the biological sample 30 is sandwiched and sealed, and the left compartment 16 and the right compartment 16 are pressed to each other due to the asserted force. As a result, the separate film 17 is broken, and the color substance 1411 and the redox substance 1412 accommodated in the left-hand compartment 16 and the right-hand compartment 16, respectively, are released and mixed. At the same time, the biological sample 30 diffuses into the permeable substrate layer 12 and further arrives at the test layer 14 because of the same asserted force. The color substance 1411 and the redox substance 1412 of the test layer 14 are mixed with the biological sample 30 to form a mixture. If the reaction of the biological sample 30, the color substance 1411, and the redox substance 1412 causes color change, it means the biological sample 30 contains occult flood.

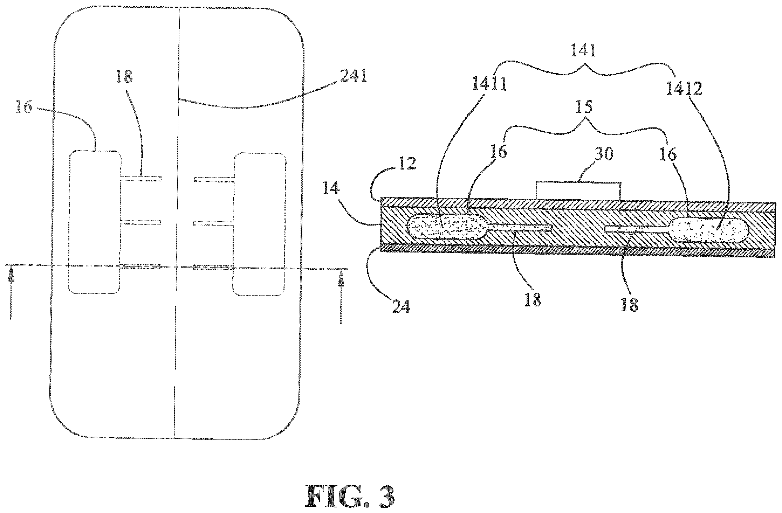

Refer to FIG 3 illustrating the test sheet for occult blood according to the third embodiment of the present invention. The left-hand part in FIG. 3 is a top view of the test sheet, and the right-hand part in FIG 3 is a side view. As shown in FIG. 3, the test sheet 10 of the third embodiment comprises a permeable substrate layer 12, a test layer 14, and an outer layer 24, which are sequentially stacked together from top to bottom, and similar to the second embodiment. For instance, the front surface of the permeable substrate layer 12 is also configured to carry the biological sample 30, and provided with at least one fold line 121. However, only one fold line 121 is shown. The test layer 14 also has at least two chambers 15 configured at two areas crossing the fold line 121. Each chamber 15 has at least two compartments 16 for accommodating the color substance 1411 and the redox substance 1412, respectively.

However, one difference between the second and third embodiment is that the third embodiment does not comprises the separate film 17. Each chamber 17 is isolated and not adjacent. The at least two chambers 15 are vertically at two sides of the fold line 121, respectively, like the left and right sides as shown in FIG. 3. In particular, the test layer 14 is provided with a plurality of connection passages 18, which are isolated and not connected to one another. Each connection passage 18 is formed by extending the corresponding chamber 15 toward the fold line 121. Specifically, the connection passage 18 has a length configured not to cross the fold line 121. When the user takes and bends the back surface of the outer layer 24 by asserting force, the test sheet 10 is folded along the fold line 121, and the permeable substrate layer 12 then sandwiches the biological sample 30. Further, the compartments 16 at the two sides of the fold line 121 are pressed by the asserted force such that the color substance 1411 and the redox substance 1412 are forced to flow out and aggregate around the fold line 121. That is, the biological sample 30 diffuses into the permeable substrate layer 12, arrives at the test layer 14 due to the asserted force, and further reacts with the mixture formed of the color substance 1411 and the redox substance 1412. If the reaction causes color change, it means the biological sample 30 possibly contain occult blood.

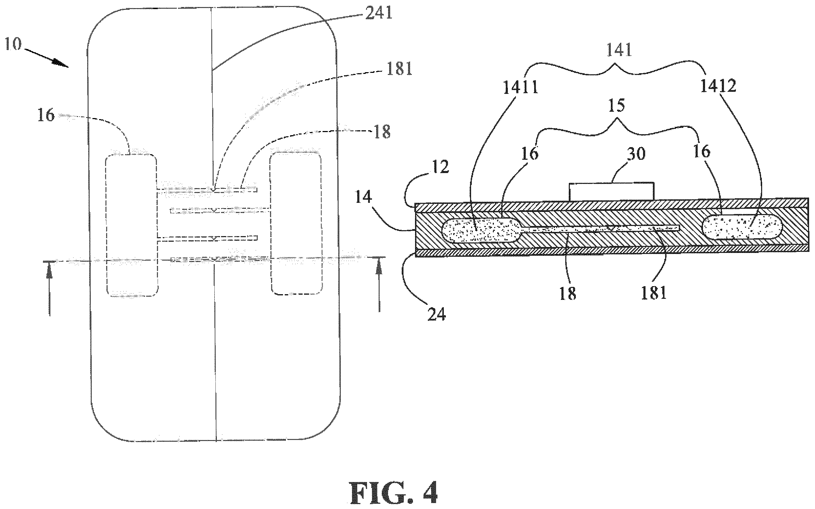

Furthermore, refer to FIG. 4 illustrating the test sheet according to the forth embodiment of the present invention. The forth embodiment is similar to the above third forth embodiment. One difference is that the connection passages 18 of the forth embodiment are configured to cross the fold line 121, and each connection passage 18 his provided with at least one notch structure 181, which is vertically aligned to the fold line 121. Thus, when the user asserts force onto the test sheet 10, the notch structures 181 of the connection passages 18 are ruptured and broken such that the color substance 1411 and the redox substance 1412 accommodated in the compartments 16 flow through the notch structures 181, and then mix up. Further, the mixture of the color substance 1411 and the redox substance 1412 diffuses and contacts the biological sample 30 around the notch structure 181 to cause the reaction. With color change caused by the reaction, it is determined whether the biological sample 30 contains occult blood.

Further, one aspect of the forth embodiment is that the compartments 16 are specifically configured to close or align the position of the user' fingers to comply with the action of the fingers such that the compartments 16 are effectively pressed and the color substance 1411 and the redox substance 1412 are forced to flow through the notch structures 181 when the fingers assert force onto the test sheet 10 to bend and fold. Also, the two compartments 16 across the fold line 121 are spaced by a specific distance to comply with the position of the fingers for improving convenience in use because too shorter or longer distance between the two compartments 16, such as too close to or far from the fold line 121, may cause the fingers to fail to assert appropriate force onto the compartments 16. As a result, the color substance 1411 and the redox substance 1412 are not effectively pressed, and the performance of occult blood test is thus adversely affected.

It should be noted that the test layer 14 of the second, third, and forth embodiments can be directly replaced by the test layer 14 of the first embodiment. In other words, the test layer 14 of the first embodiment may also employ the compartment 16 of the chamber 15 to accommodate the test agent 141 to replace the technical characteristic of the test agent 141 evenly distributed in the test layer 14 as shown in FIG. 1.

Obviously, one feature of the present invention is that the fold line is employed to help the user to easily fold test sheet so as to keep sanitation by sandwiching the biological sample. Further, the biological sample is pressed to diffuse and contact the test agent while the fold line folded. Thus, through the back surface of the outer layer, the user easily examines if the biological sample mixed with the test agent for reaction demonstrates color change, indicating the biological sample contains occult blood. If the biological sample containing occult blood is feces, it is very like to develop colorectal cancer. Therefore, the test sheet of the present invention provides a function of examining occult blood by easily and simply wiping and folding the test sheet to preliminarily identify occult blood without any pre-treatment or post-treatment for the biological sample. In particular, the user would not feel any discomfort, and the test sheet is very sanitary.

Another feature of the present invention is that the overall operation is simple for ordinary people to perform alone at home without any specific assistance by medical personnel, thereby possessing industrial utility and market competitiveness.

Although the present invention has been described with reference to the preferred embodiments thereof, it is apparent to those skilled in the art that a variety of modifications and changes may be made without departing from the scope of the present invention which is intended to be defined by the appended claims.

## Claims

1. A test sheet for occult blood, comprising:
a permeable substrate layer with water permeability, having a front surface for carrying a biologic sample and a back surface;
a test layer with translucency and water permeability, having a front surface and a back surface, containing a test agent in at least one area of the test layer; and
an outer layer with transparency and water impermeability, attached to the back surface of the permeable substrate layer and the back surface of the test layer,
wherein one side of the permeable substrate layer is connected to one corresponding side of the test layer, at least one fold line is formed on a front surface of a connection of the permeable substrate layer and the test layer, and the biologic sample on the front surface of the permeable substrate layer diffuses into the test layer after the at least one fold line is folded, and is further mixed with the test agent to proceed a predetermined reaction to cause color change for examining if the biologic sample contains occult blood.

2. The test sheet as claimed in claim 1, wherein the permeable substrate layer comprises at least one of chemical fiber, plant fiber, animal fiber, and mineral fiber, and the test layer comprises at least one of plant fiber, nitride fiber, polyethylene (PE), polypropylene (PP), cellulose acetate fiber, polyvinylchloride (PVC) and polyvinylidenechloride (PVDC).

3. The test sheet as claimed in claim 2, wherein the chemical fiber comprises at least one of polyester, nylon, spandex, acrylate, and cellulose acetate fiber, the animal fiber comprises at least one of animal hair, silk fiber, and spider fiber, and the mineral fiber comprises asbestos.

4. A test sheet for occult blood, comprising:
a permeable substrate layer with water permeability, having a front surface for carrying a biologic sample and a back surface, at least one fold line formed on a middle region of the front surface for folding;
a test layer with translucency and water permeability, having a front surface and a back surface, the front surface of the test layer attached to the back surface of the permeable substrate layer, the test layer comprising at least one chamber, the chamber having at least two compartments for accommodating a test agent, the at least two compartments separated each other by a separate film, the separate film vertically aligned to the fold line, the test agent comprising a color substance and a redox substance accommodated in the two different compartments, respectively; and
an outer layer with transparency and water impermeability, attached to the back surface of the test layer,
wherein the redox substance possesses a function of proceeding a redox reaction with an iron-based matter for decomposing and releasing free electrons, the color substance possesses a function of receiving the free electrons released from the redox substance to cause color change, the biological sample is sandwiched by the front surface of the permeable substrate layer after the fold line is folded, the biological sample diffuses into the test layer, the separate film is broken and releases the color substance and the redox substance accommodated in the at least two compartments after the fold line is folded, and the color substance and the redox substance contact and react with the biological sample to cause color change for examining if the biologic sample contains occult blood.

5. The test sheet as claimed in claim 4, wherein the permeable substrate layer comprises at least one of chemical fiber, plant fiber, animal fiber, the chemical fiber comprises at least one of polyester, nylon, spandex, acrylate, and cellulose acetate fiber, the animal fiber comprises at least one of animal hair, silk fiber, and spider fiber, the mineral fiber comprises asbestos, and the test layer comprises at least one of plant fiber, nitride fiber, polyethylene, polypropylene, cellulose acetate fiber, polyvinylchloride and polyvinylidenechloride.

6. The test sheet as claimed in claim 5, wherein the iron-based matter comprises an iron-based substance of hemoglobin in blood, the redox substance comprises at least one of hydrogen peroxide and sodium percarbonate, and the color substance comprises at least one of o-toluidine, phenolphthalein, pyramidon, guaiac, guaiac gum, tetramethylbenzidine, and organic alcohol.

7. A test sheet for occult blood, comprising:
a permeable substrate layer with water permeability, having a front surface for carrying a biologic sample and a back surface, at least one fold line formed on a middle region of the front surface for folding;
a test layer with translucency and water permeability, having a front surface and a back surface, the front surface of the test layer attached to the back surface of the permeable substrate layer, the test layer comprising at least one chamber, the chamber having at least two compartments for accommodating a test agent, the at least two compartments vertically provided on two sides of the fold line, the test agent comprising a color substance and a redox substance accommodated in the two different compartments, respectively, the test layer provided with a plurality of connection passages configured not to cross one another, each connection passage formed by extending the corresponding chamber toward the fold line; and
an outer layer with transparency and water impermeability, attached to the back surface of the test layer,
wherein the redox substance possesses a function of proceeding a redox reaction with an iron-based matter for decomposing and releasing free electrons, the color substance possesses a function of receiving the free electrons released from the redox substance to cause color change, the biological sample is sandwiched by the front surface of the permeable substrate layer after the fold line is folded, the biological sample diffuses into the test layer, the color substance and the redox substance accommodated in the least two compartments are released and forced to flow and accumulate around the fold line after the fold line is folded; and the color substance and the redox substance contact and react with the biological sample to cause color change for examining if the biologic sample contains occult blood.

8. The test sheet as claimed in claim 7, wherein the permeable substrate layer comprises at least one of chemical fiber, plant fiber, animal fiber, the chemical fiber comprises at least one of polyester, nylon, spandex, acrylate, and cellulose acetate fiber, the animal fiber comprises at least one of animal hair, silk fiber, and spider fiber, the mineral fiber comprises asbestos, the test layer comprises at least one of plant fiber, nitride fiber, polyethylene, polypropylene, cellulose acetate fiber, polyvinylchloride and polyvinylidenechloride, the iron-based matter comprises an iron-based substance of hemoglobin in blood, the redox substance comprises at least one of hydrogen peroxide and sodium percarbonate, and the color substance comprises at least one of o-toluidine, phenolphthalein, pyramidon, guaiac, guaiac gum, tetramethylbenzidine, and organic alcohol.

9. The test sheet as claimed in claim 7, wherein the connection passage has a length configured not to cross the fold line.

10. The test sheet as claimed in claim 7, wherein the connection passage has a length configured to cross the fold line, the connection passage is provided with at least one notch structure configured for vertically aligning the fold line, the at least one notch structure is broken after the fold line is folded, the color substance and the redox substance accommodated in the at least two compartments are released through the at least one notch structure to diffuse, contact and react with the biological sample around the at least one notch structure.
